Europäisches Patentamt

European Patent Office

Office européen des brevets

(19)

(11) Numéro de publication : **0 352 146 B1**

## FASCICULE DE BREVET EUROPEEN

(12)

(45) Date de publication du fascicule du brevet :
30.09.92 Bulletin 92/40

(51) Int. Cl.⁵ : **A61K 35/78**

(21) Numéro de dépôt : **89401191.5**

(22) Date de dépôt : **26.04.89**

(54) **Régulateur biologique extrait de Gingko Biloba, actif dans diverses pathologies.**

(30) Priorité : **19.07.88 FR 8809738**

(43) Date de publication de la demande :
**24.01.90 Bulletin 90/04**

(45) Mention de la délivrance du brevet :
**30.09.92 Bulletin 92/40**

(84) Etats contractants désignés :
**AT BE CH DE ES GB GR IT LI LU NL SE**

(56) Documents cités :
**EP-A- 0 086 315**
**FR-A- 2 132 761**

(73) Titulaire : **ABRAXAS BIO LABS S.A.**
**231, Val des Bons Malades**
**L-2121 Luxembourg-Kirchberg (LU)**

(72) Inventeur : **Beljanski, Mirko**
**46, boulevard de Port Royal**
**F-75005 Paris (FR)**

(74) Mandataire : **Pinguet, André**
**Cabinet de Proprieté Industrielle CAPRI 28**
**bis, avenue Mozart**
**F-75016 Paris (FR)**

## Description

La présente invention a pour objet un nouveau produit, extrait des feuilles de l'arbre Gingko Biloba et à usage de régulateur biologique actif dans diverses pathologies. Pour la commodité de l'exposé, ce produit sera appelé BIOPARYL dans la description et les revendications. Ce n'est pas une marque, c'est un nom proposé pour désigner ce produit.

## Description de l'art antérieur

Le document EP-A-0 086 315 décrit un procédé d'extraction d'un produit vaso actif à partir des feuilles vertes de Gingko Biloba. Le procédé d'extraction comporte les étapes suivantes :
– épuisement par un solvant hydrocarboné,
– collecte de l'insoluble,
– extraction par un solvant cétonique aqueux,
– séparation de la phase organique,
– addition de sulfate d'ammonium et d'une cétone aliphatique insoluble dans l'eau,
– séparation de la phase cétonique,
– concentration,
– dilution par un alcool aqueux,
– addition d'un acide minéral ou organique aqueux,
– traitement de cette solution acide par un polymère insoluble de vinylpyrrolidone préalablement purifié,
– séparation du polymère par filtration ou centrifugation,
– neutralisation du filtrat ou surnageant, et
– séchage.

Le document FR-A-2 132 761 décrit un autre procédé d'extraction d'un produit vaso actif à partir des feuilles vertes de Gingko Biloba. Le procédé d'extraction comporte les étapes suivantes :
– extraction au moyen d'une cétone ou d'un alcool aliphatique inférieur, à une température d'environ 40 à 100°C,
– extraction de l'extrait obtenu au moyen d'un solvant lipophile non miscible à l'eau à une température d'environ 15 à 50°C,
– réaction de l'extrait hydraté avec du sulfate d'ammonium,
– extraction de la solution au moyen de la méthylcétone,
– évaporation de l'extrait de méthylcétone,
– dilution dans un alcool aliphatique inférieur éventuellement hydraté,
– traitement de la solution alcoolique ainsi obtenue avec un composé de plomb, ou un polyamide pulvérulent à poids moléculaire élevé,
– élimination respectivement du précipité ainsi formé ou des résidus solides,
– concentration du filtrat ou de la solution alcoolique.

## Brève description de l'invention

Le maintien cellulaire puis la division de la cellule exigent la reproduction du génome (ADN), puis la transcription de celui-ci en divers ARN dont les ARN-messagers, ces derniers dirigeant à leur tour la synthèse des protéines. L'extrême complexité du milieu biologique permet aux enzymes présentes d'assurer l'ensemble de ces fonctions.

La pathologie peut résulter d'altérations ou de dégradations des éléments en cause dans l'une ou l'autre de ces étapes. En outre, des facteurs endogènes ou exogènes peuvent également affecter le déroulement de ces diverses étapes. Mais inversement, une dérégulation, un état pathologique peuvent être améliorés par l'introduction dans le système biologique d'entités agissant directement ou indirectement, mais toujours de façon sélective, sur l'ADN, sur les ARN ou les enzymes.

Un matériel a été préparé et nommé "BIOPARYL". Il joue un rôle important dans la régulation de certaines enzymes, les ribonucléases en particulier mais d'autres également, enzymes impliquées dans les mécanismes fondamentaux du fonctionnement des gènes. Il agit à différents niveaux cellulaires, toujours de façon sélective sur ce qui est pathologique, enzymes dérégulées, ADNs de cellules lesées ou même cancéreuses. Son mode de préparation est original ainsi que son champ d'application.

Bioparyl est obtenu à partir de feuilles dorées de l'arbre Gingko biloba, récoltées à la fin de l'automne. C'est une préparation standardisée comportant plusieurs étapes : extraction du principe contenu dans les feuilles par plusieurs heures d'ébullition en présence d'eau. Concentration de l'extrait. Hydrolyse à chaud, neutralisation partielle, centrifugation et fractionnement sur colonne de chromatographie. Seule une sous fraction de l'extrait initial constitue le Bioparyl. Le Bioparyl est dépourvu d'effets toxiques ou secondaires , tant au niveau cellulaire que chez l'animal. Il peut être utilisé durant de longues périodes afin d'améliorer diverses pathologies. Il est exempt des effets sur la tension artérielle habituels aux autres produits préparés par d'autres méthodes à partir des feuilles du même arbre.

Le Bioparyl est très efficace dans le traitement ou dans la prévention de la fibrose de divers tissus ou organes survenue soit sans cause connue soit suite à différents traitements : rayons, médicaments (bleomycine).Il peut guérir des fibroses déjà installées par ces agents . Il possède un certain pouvoir spécifique contre les cellules tumorales . Il normalise aussi le taux des gamma-globulines trop élevé , s'accumulant dans certaines maladies inflammatoires, maladies auto-immunes ou maladies virales, tel le Syndrome d'Immuno-Déficience (SIDA).

Chez les patients atteints de maladies malignes (cancers solides, cancers du sang ou de la lymphe ),on détecte pratiquement toujours des anomalies quantitatives et/ou qualitatives de l'activité des ribonucléases (RNases).Le Bioparyl normalise le le fonctionnement et le taux de ces enzymes, ce qui a pour conséquences d'améliorer l'état général du malade et d'entraver l'apparition d'éléments nécessaires au développement de la pathologie.

## Illustrations

Fig.1- Activité in vitro des RNases du plasma de personnes saines ou porteuses de cancer sur les ARN radioactifs ribosomique et de transfert (ARN-r et ARN 4 S).

Fig.2 et 3- Effet du BIOPARYL sur les activités des RNases du plasma de malades porteurs de différentes pathologies.

Fig.4- Absence d'effet du BIOPARYL sur les RNases du plasma des sujets sains.

Fig.5- Action du fer sur l'activité des RNases chez un sujet atteint de cancer et chez une personne saine . Action du BIOPARYL.

Fig.6- Action normalisatrice du BIOPARYL sur les RNases plasmatiques chez les sujets porteurs de cancers.

Fig.7- Activité des RNases de la peau humaine fibrosée.Action du fer et du BIOPARYL.

Fig.8- Stimulation par le BIOPARYL de la synthèse in vitro de l'ADN de la peau saine. Belle action inhibitrice sur celle du mélanome, et

Fig.9- Inhibition de la synthèse d'ADN des cellules cancéreuses.

En automne , juste au moment de leur chute , les feuilles dorées de l'arbre Gingko biloba (aussi quelquefois appelé Ginkgo) sont récoltées. Elles peuvent être soit congelées, soit séchées .

Cinq à sept kilos de feuilles congelées (ou environ 2,5 -3 kg de feuilles sèches) sont recouvertes d'eau du robinet (environ 7-8 litres) et l'ensemble est porté à ébullition pendant 3 à 4 heures. Le liquide est prélevé puis concentré à 1 litre ( une deuxième ébullition des feuilles est pratiquée et le liquide provenant de cette seconde extraction servira pour la première extraction d'un nouveau lot de feuilles ).

Pour 1 litre de liquide concentré , l'acide chlorhydrique concentré est additionné afin d'avoir une concentration d'acide de 1 N finale . Le mélange est alors porté à 100°C pendant 30 min . Lorsque la température est redescendue à environ 50°C , le pH est ramené à environ 3 ou 4 par addition de K0H ou Na0H). Une centrifugation rapide ( 5-7 min à 6000 tours/min.) est pratiquée. Une quantité très importante de matériel "hydrolysé" insoluble est alors éliminée. Le surnageant (liquide brun-rouge) est seul conservé et utilisé soit directement soit congelé. Ce matériel est appelé ZAC.

200 - 300 g de ZAC (environ 300-400 ml , poids sec déterminé sur 0.5 ml ) sont déposés au sommet d'une colonne de Sephadex (R)G-25 fine (70 cm x 7 cm) préalablement gonflé et équilibré en eau déminéralisée.

L'élution s'effectue à 'aide d'eau déminéralisée, du début de l'élution à la fin de la partie fluorescente blanc-jaune (visible en lumière ultraviolette) ; les éluats sont réunis et concentrés soit par distillation soit par ébullition sous air comprimé , ce qui est un moyen rapide. Lorsque l'éluat est concentré à environ 1 litre , le pH est ajusté à environ 7,0 et la quantité de matériel est déterminée par pesée poids sec d'une quantité aliquote.

Le poids total divisé par 30 donnera le nombre de chaque malade pouvant utiliser pendant 30 jours (1 mois) 0.5 g x 2 chaque jour de BIOPARYL. Soit A ce nombre de malades .

Le liquide est alors concentré à nouveau à environ 500 ml puis adsorbé sur de la poudre de cellulose (Whatman (R) CF1) et séché dans un four (60 - 80°C) pendant plusieurs heures. Le matériel doit être mélangé de temps à autre afin d'éviter la formation de grumeaux .

Une fois séchée, la poudre est pulvérisée, pesée et ce poids divisé par le chiffre A donne le poids du mélange à répartir pour un mois en 60 gélules (2 gélules/jour , soit 2 x 0.5 gr de BIOPARYL/jour) .

L'éluat de la colonne, concentré à 500 ml (pH 7.0) est soluble dans l'eau. Ce BIOPARYL, préparé selon la technique qui vient d'être décrite a été utilisé dans toutes les expériences décrites ci-dessous. Cependant , pour des réactions fines, la préparation est préalablement diluée, recentrifugée et filtrée sur millipore avant que son poids soit à nouveau déterminé.

Les composés se trouvant dans les feuilles de Gingko Biloba ont été étudiés par divers auteurs . Cependant aussi bien la méthode de préparation que les applications diffèrent totalement. Elle diffère dans le choix du matériel (feuilles dorées et non vertes), dans le liquide d'extraction (ici de l'eau et non pas des solvents comme toujours décrit dans la littérature), par l'hydrolyse qui élimine beaucoup de composés non souhaitables, par la centrifugation après que le pH ait été ajusté, qui élimine encore une grande partie de matériel puis par la sélection sur colonne. Ici encore, une importante partie du matériel initial est éliminé de ce qui constituera le BIOPARYL (lorsqu'en fait la colonne de Séphadex est lavée à la soude (0,1 N 300 ml) puis à l'eau jusqu'à pH neutre on constate qu'une partie très importante de matériel était restée sur la colonne. Ainsi lavée la colonne peut reservir).

Des extraits de feuilles vertes de Gingko Biloba additionnés d'autres substances, sont dans le commerce et soit destinés à modifier la circulation artérielle, capillaire ou veineuse, soit à améliorer la circulation dans le cerveau. Toutes ces préparations présentent des contre-indications qui semblent ne pas exister pour le Bioparyl dont un dosage à des quantités beaucoup plus importantes (jusqu'à 2 g/jour pendant des semaines) n'a pas permis de déceler la moindre variation ou altération de l'état du patient.

La difficulté d'analyse des divers composés se trouvant dans les feuilles de l'arbre Gingko biloba est bien connue .De nombreuses firmes pharmaceutiques s'y sont en vain essayées et vendent une "composition standardisée"d'extrait de feuilles de Gingko biloba. C'est pourquoi nous insistons tout particulièrement sur le fait que notre technique de préparation standardisée diffère totalement de celle utilisée par les autres firmes pharmaceutiques et que les applications sont elles aussi totalement différentes . C'est d'autant plus important qu'à l'heure actuelle il n'existe aucun autre procédé de lutter contre la fibrose ou de réguler la synthèse de diverses enzymes ou protéines , particulièrement importantes dans des maladies qui , actuellement , occupent le devant de l'actualité. En outre et bien que notre extrait soit entièrement démuni d'effets secondaires , le Bioparyl possède un effet anticancéreux qui , sans être d'une très puissante activité , contribue néammoins à maintenir et recouvrir un état de santé déficient chez les animaux porteurs de cancers.

Exemple 1 : Action du Bioparyl sur les RNases

Les personnes atteintes de divers cancers présentent très généralement des anomalies au niveau de l'activité des RNases de leur plasma. Le Bioparyl possède un fort effet régulateur, in vitro comme in vivo , c'est à dire qu'il ramène l'activité des RNases du plasma à des activités proches de celles trouvées chez les personnes saines ; En fonction du temps, un malade prenant régulièrement du Bioparyl normalise l'activité de ses ribonucléases; L'effet reste après que le traitement par le Bioparyl soit suspendu.

Le sang périphérique est prélevé sur citrate de sodium (10 mg pour 5 ml de sang) afin d'éviter la coagulation du sang sans interférer avec l'activité des enzymes. Après centrifugation, le plasma est délicatement prélevé, servant de source de ribonucléase.Le plasma est utilisé après dilution (10 fois) à l'aide de tampon Tris-Hcl $10^{-3}$M, pH 7,60 et le taux de protéines déterminé (Biuret).

Le Bioparyl dissous dans de l'eau distillée (pH 7.0, 5 mg/ml) est utilisé (0.01 à 0.05 ml). Le milieu d'incubation (0,15 ml final) contient : 0,05 ml de tampon Tris-Hcl $10^{-2}$M, (pH 7,6 ) ; 0,05 ml de $^{3}$H-ARN (ARN-r ou ARN 4 S, 100 µg, 15000 CPM) ; 0,01 à 0,05 ml de la dilution au 1/10 de plasma . Après incubation à 36°C pendant 10 minutes (bain marie), conditions généralement nécessaires mais pouvant varier en cas de nécessité, la réaction est arrêtée par addition de 3 ml d'acide trichloracétique (ATC) à 5%, les tubes sont refroidis afin d'assurer une bonne précipitation des protéines et de l'ARN ($^{3}$H) non dégradé. Le précipité filtré sur millipore de verre (GF/C (R), Whatman (R)) est lavé par l'ATC 5% puis par l'alcool ethylique à 95°, les filtres sont séchés et la radioactivité est déterminée dans un compteur à scintillation. Les résultats sont exprimés en pourcent d'ARN radioactif non dégradé (la radioactivité de l'ARN intact étant prise comme 100%).

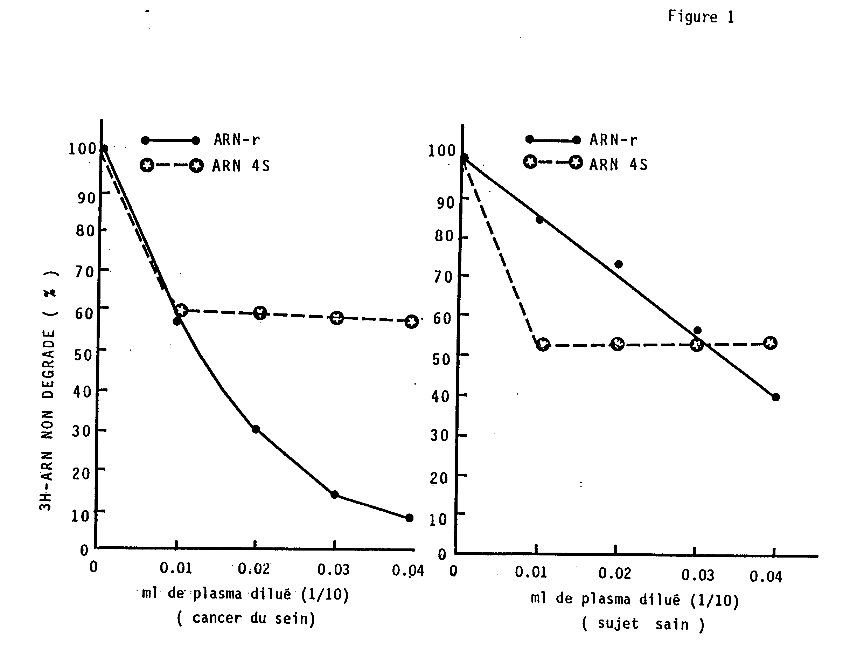

La figure 1 montre que la RNase du plasma d'une malade atteinte d'un cancer du sein dégrade fortement l'ARN ribosomique (ARN à longue chaine), alors qu'une telle dégradation ne s'observe pas avec l'ARN 4 S (ARN à courte chaine). Sur la figure 1, on voit que chez le sujet sain l'ARN à longue chaine est beaucoup moins dégradé que par l'enzyme du sujet cancéreux.

D'une façon générale, les activités des RNases des sujets porteurs de cancers sont fortement augmentées , ce qui stimule le catabolisme, affaiblit le malade et participe au maintien de l'état cancéreux. Dans l'état actuel des choses, on ne peut dire si cette augmentation des RNases est dûe à un taux plus élevé de ces enzymes (suractivation des gènes correspondants) ou à la présence d'un métal par exemple , accélérant ces activités.

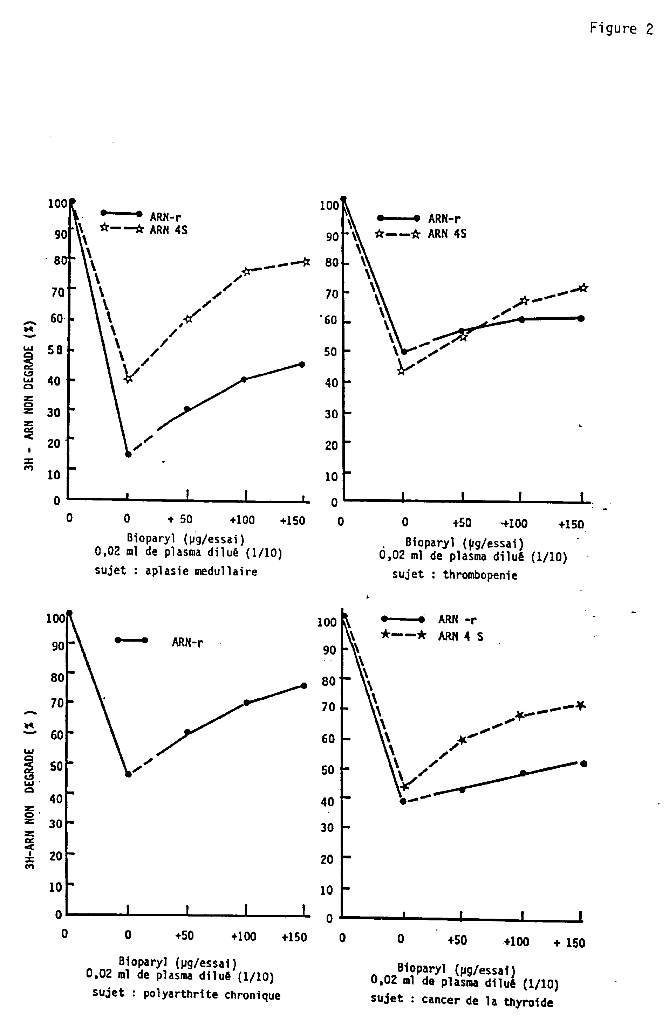

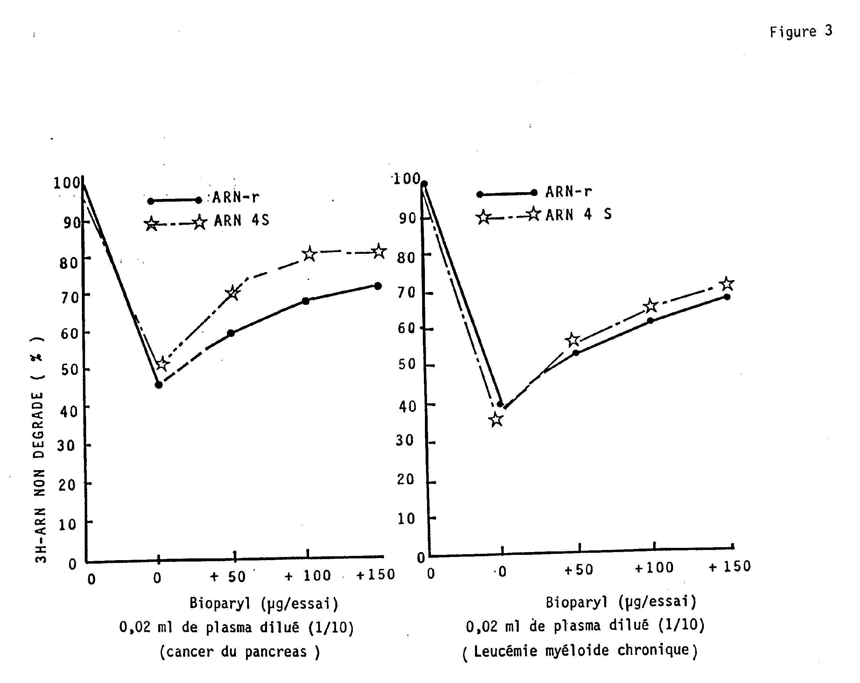

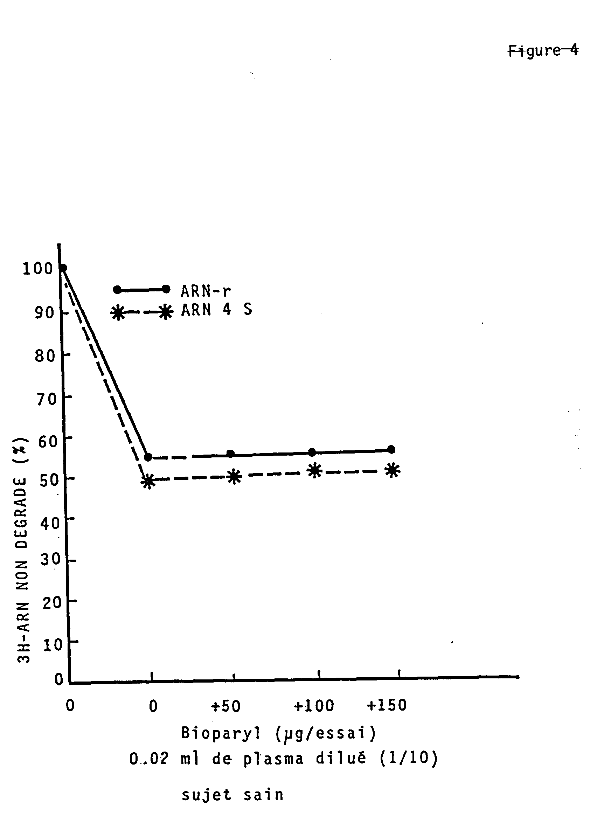

Le Bioparyl inhibe in vitro l'activité des RNases trop actives du plasma des malades (Fig.2 et 3 ),mais il est sans action sur la RNase du plasma des personnes saines (Fig.4). Ceci indique soit que les enzymes sont différents, soit qu'il existe des co-facteurs particuliers permettant la sélectivité d'action du Bioparyl, phénomène extrêmement important.

Exemple 2 .Effet du fer et rôle du Bioparyl sur l'activité in vitro des RNases.

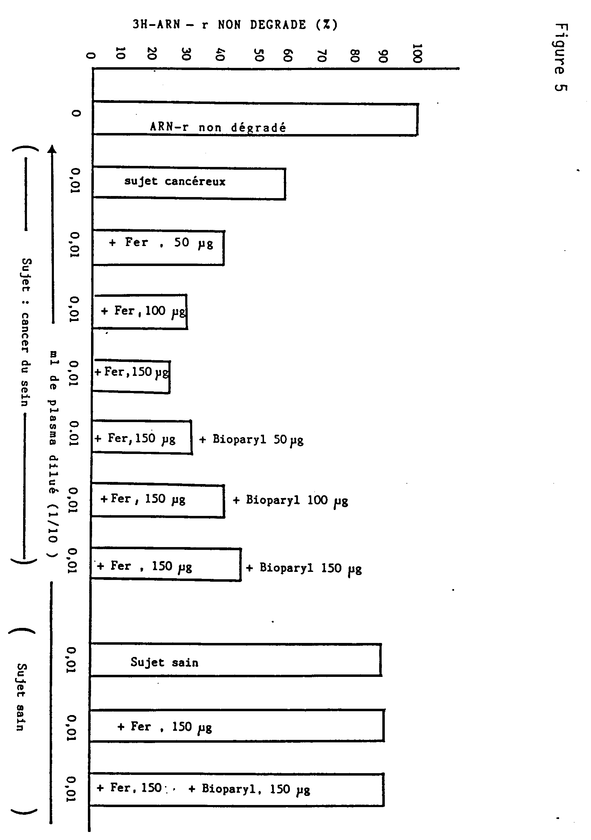

Le fer ferrique accélère fortement l'activité in vitro des RNases des sujets atteints de cancers ( malades atteints de leucémies, de cancers solides (foie en particulier), de maladies auto-immunes (polyarthrites, thrombopénies) et dans certaines pathologies virales, SIDA par exemple ) . Mais le fer est sans action sur l'activité in vitro des RNases du plasma des sujets sains. Figure 5 . On sait que les RNases peuvent fournir des ARN amorceurs nécessaires à la réplication des ADN et que les amorceurs acceptés par les ADN des tissus sains ne sont pas les mêmes que les amorceurs nécessaires à la réplication des ADN de tissus cancéreux. De ce seul fait, la régulation des RNases est un phénomène capital en cancérologie et en virologie. Le Bioparyl, par son aptitude à réguler ce qui est perturbé et cela seulement, même en présence de fer, est un agent de normalisation extrêmement novateur et important.

Exemple 3 : Normalisation in vivo des RNases plasmatiques d'un sujet atteint de cancer et prenant régulièrement du Bioparyl.

Dans les cas de cancers solides ou chez les leucémiques (leucémie myéloide chronique en particulier) soumis ou non à la chimiothérapie, le Bioparyl, administré per os (1 gr/jour chaque jour) corrige, en fonction du temps, l'activité perturbée au départ des RNases du sang circulant et rapproche leurs valeurs de celles observées chez les sujets sains.

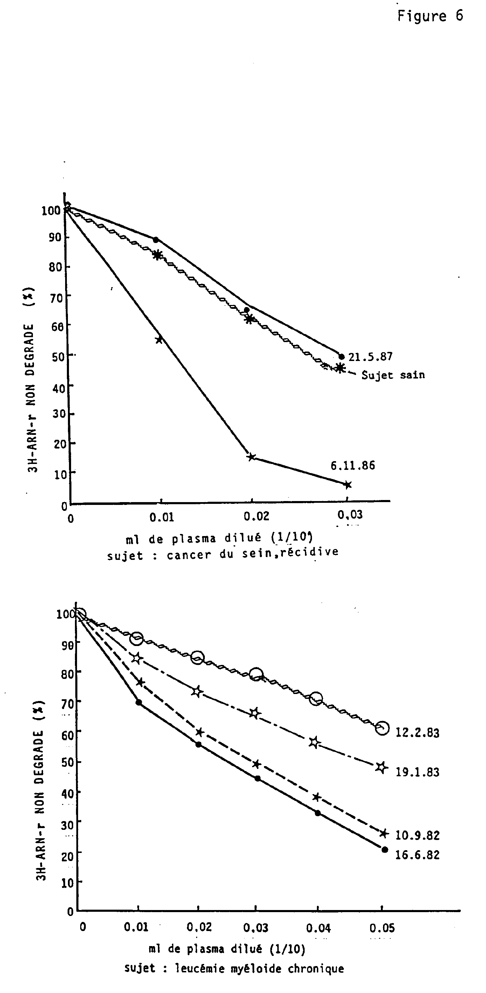

La Figure 6 montre en fonction du temps la normalisation de l'activité des RNases plasmatiques chez deux sujets porteurs de deux types différents de cancer (arrêt traitement 3 jours avant l'anayse).

Exemple 4 : fibrose radique . Etude in vitro .

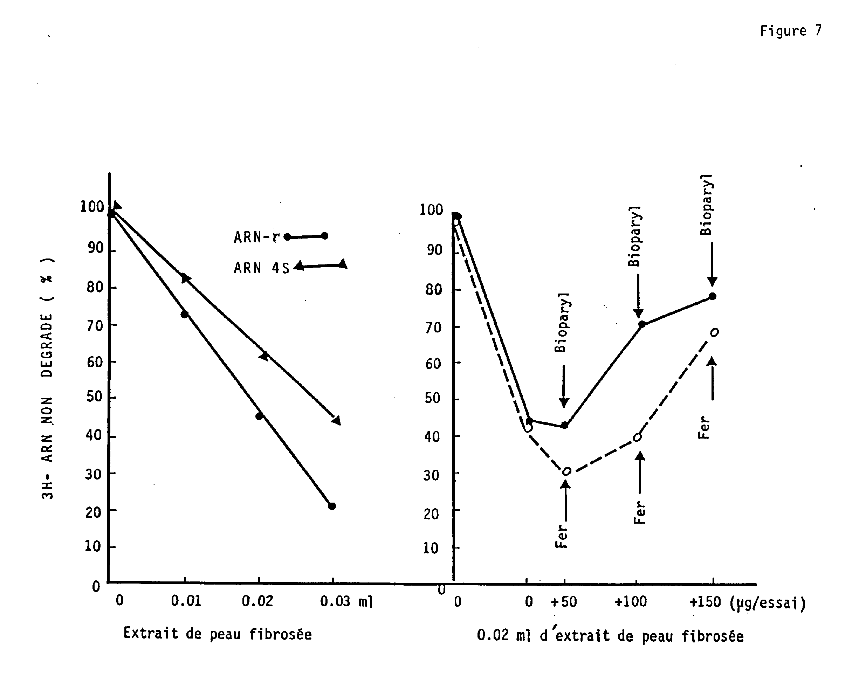

Un prélèvement de peau fibrosée par les rayons a été broyé dans un mortier (4°C) en présence de tampon Tris-HCl 0,02 M contenant 0,06 M de KCl et 0,001 M de MgCl$_2$. Volume final : 1,5 ml . L'activité des RNases contenues dans cet extrait a été testée vis à vis de l'ARN 4 S et vis à vis de l'ARN ribosomique (ARN-r); 0,01 ; 0,02 ; 0,03 et 0,04 ml d'extrait enzymatique sont incubés en présence d'ARN radioactif $^3$H-ARN-r ou $^3$H 4S ARN. Temps d'incubation :15 min à 36°C. Les résultats sont illustrés dans la figure 7 . La dégradation des ARN marqués est linéaire en fonction de diverses concentrations d'extrait. L'ARN-r est plus rapidement dégradé que l'ARN 4 S. Le Bioparyl inhibe fortement mais non totalement la dégradation in vitro de l'ARN-r.
Le fer ferrique (FeCl$_3$) à faible concentration stimule la dégradation de l'ARN-r mais à fortes concentrations il l'inhibe. Pour des raisons d'éthique , nous n'avons pas utilisé la peau normale d'un sujet humain . Mais le Bioparyl n'agit pas sur la RNase de la peau normale de jeune agneau .

Action du Bioparyl sur l'ADN de la peau

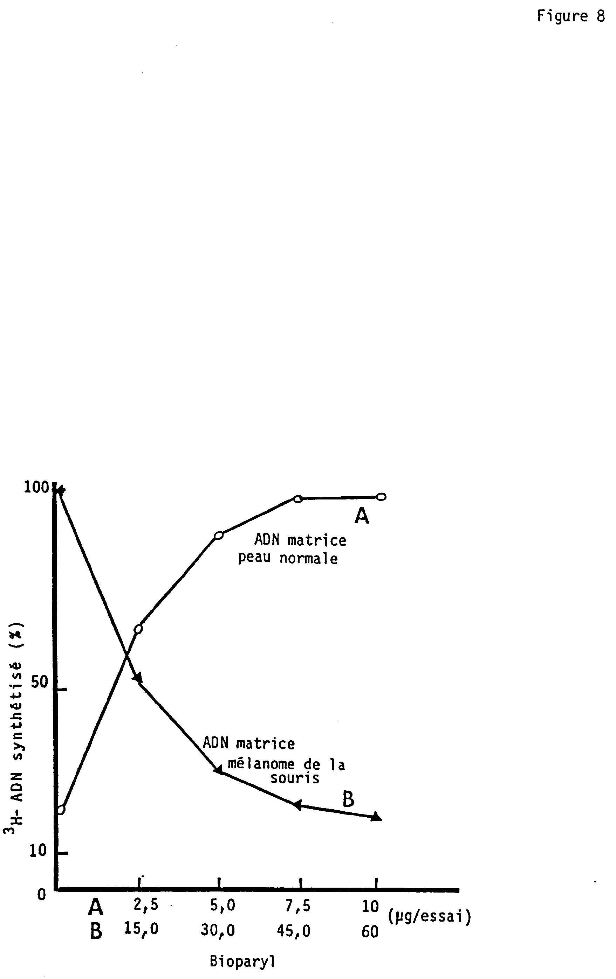

Le Bioparyl stimule la synthèse in vitro de l'ADN de la peau saine de jeunes moutons ainsi que celle d'ADN isolés de la peau de souriceaux.
L'ADN de la peau saine d'animaux jeunes a été isolée par le procédé décrit dans Experimental Cell Biol. 49 : 220-231 (1981) qui donne également la methode utilisée pour la synthèse in vitro de l'ADN . La figure 8 montre que le Bioparyl stimule fortement la réplication de l'ADN de la peau saine . Il est tout à fait remarquable d'observer la très belle inhibition de la synthèse in vitro de l'ADN isolé du mélanome de la souris en présence de très fortes concentrations de Bioparyl ( mélanome B 16 F 10 aimablement mis à notre disposition par Mme Poupon de l'Institut Gustave Roussy ).

Action in vivo du Bioparyl sur la fibrose .

Les lésions post-radiques ont été traitées chez des sujets ayant subi la radiothérapie , par le Bioparyl . La dose orale était de 0,5 g x 2 / jour pendant plusieurs semaines .L'efficacité est certaine surtout dans les fibroses cutannées, musculaires (péricarde du coeur) ou muqueuses. On obtient 83 % de réponses objectives dans les fibroses cutanéo-muqueuses post-radiques. Les péricardites fibrosantes peuvent être efficacement guéries par le Bioparyl. Lorsque la fibrose date de "moins d'un an", 71% de réponses objectives sont obtenues. L'effet dose n'a pas encore été déterminé.

Exemple 5 Le Bioparyl corrige le taux des γ- et immuno-globulines élevé chez les porteurs du virus du SIDA ou chez les malades atteints de maladies auto-immunes.

Certaines pathologies (SIDA, polyarthrites, scléroses ou autres maladies) induisent des taux anormalement élevés de diverses immuno-globulines (IgM, IgG, etc.), ce qui est néfaste à l'équilibre immunitaire. La prise de Bioparyl par voie orale (1 gr/jour chaque jour) permet d'observer en 3-5 semaines de traitement une baisse du taux des globulines. On constate une amélioration clinique immédiate qui se poursuit tout au long du traitement par le Bioparyl. Les résultats sont illustrés dans le tableau 1, qui présente le rôle du Bioparyl dans la normalisation du taux des gamma-globulines et immuno-globulines dans deux cas de maladies auto-immunes et dans un cas de SIDA.

TABLEAU I

<u>Exemple I : malades atteints de maladies auto-immunes</u>

Immunoglobulines M (IgM) gramme/litre

| Jours | <u>Malade A</u> | <u>Malade B</u> |
|---|---|---|
| j.0 Bioparyl | 5 | 9   (N = 0,65-2,10) |
| j.40 | 2 | - |
| j.60 | - | 3 |

<u>Exemple II : sujet sida-positif avant et après traitement par le Bioparyl</u>

| Jours | Taux de $\gamma$-globulines % | taux d'IgG % | taux d'albumine % |
|---|---|---|---|
| j.0 Bioparyl | 27,7 (N = 12-18) | 24.5 (N = 7-14) | 49 (N = 52-60) |
| j.15 | 21 | 21.8 | 62 |
| j.25 | 20 | 19.5 | 60 |

EP 0 352 146 B1

EP 0 352 146 B1

Exemple 6 Inhibition de la synthèse d'ADN des cellules cancéreuses.

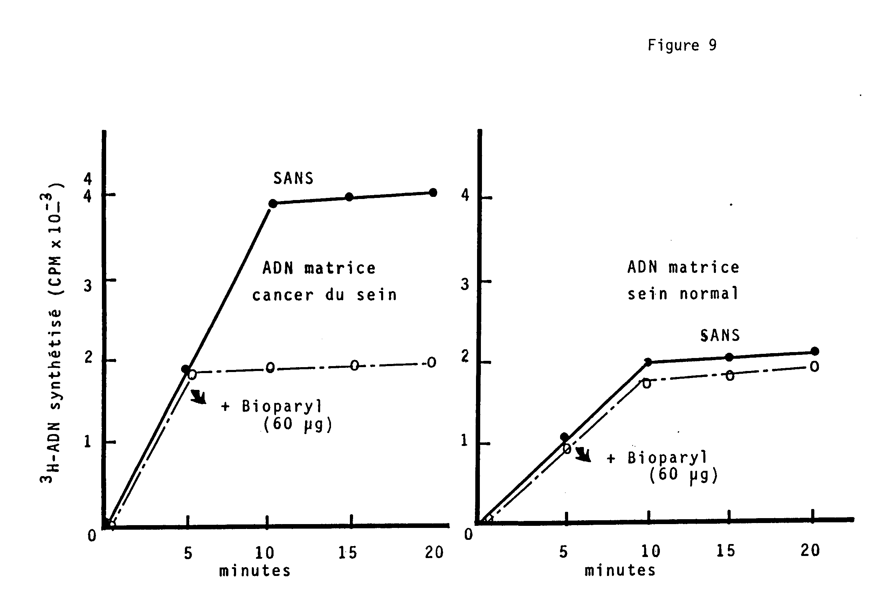

Dans les conditions in vitro, le Bioparyl agit seulement sur la synthèse de l'ADN des cellules cancéreuses (cancer du sein, de l'ovaire et du poumon humains) sans affecter, dans les mêmes conditions que celle de l'ADN des tissus sains (sein, poumon, rate ou moelle osseuse des humains). L'initiation et l'élongation de la formation d'ADN cancéreux sont arrêtées par le Bioparyl, alors que celles de l'ADN normal ne le sont pas. Figure 9. Ceci se démontre en introduisant le Bioparyl soit au départ soit après que la synthèse ait démarré, comme ceci est décrit en détail dans ma demande de brevet français n°. 88-08434 du 23 juin 1988 (FR-A-2633182, EP-A-0 352147).

In vivo chez les souris : 60 souris BALB C (30 ♂ et 30 ♀ de 18 gr) porteuses de cellules tumorales (lymphome YC8) et 60 souris Cdl Swiss (30 ♂ et 30 ♀) porteuses de cellules tumorales (Ascite d'Ehrlich) (4 à 6000 cellules par voie i.p. 48 h avant le début du traitement) reçoivent Bioparyl deux fois par jour (2 x 3 mg/jour pendant 8 jours consécutifs, voie i.p.). Les lots témoins reçoivent, au lieu de Bioparyl, une solution isotonique de NaC1. Le tableau 1 présente l'inhibition de la multiplication des cellules cancéreuses chez la souris sous l'effet du Bioparyl (ascite d'Ehrlich). Les résultats exposés dans le tableau II révèlent un certain effet anticancéreux du Bioparyl qui toutefois, chez l'homme et per os, n'est pas suffisant.

8

TABLEAU II  <u>Survie de souris ♂ Swiss porteuses des cellules ascitiques d'Ehrlich</u>
<u>et traitées par Bipoparyl</u>

Survie (jours)            Souris non traitées                   Souris traitées par Bioparyl

0    ....................    10/10    ....................    10/10
21   ....................    0/10     ....................    6/10
60   ....................    −        ....................    4/10
90   ....................    −        ....................    4/10

Bioparyl : 2 x 3 mg/j pendant 8 jours consécutifs (voie i.p.)


<u>Survie des souris ♀ Swiss porteuses des cellules ascitiques d'Ehrlich</u>
<u>et traitées par Biopary</u>

Survie (jours)            Souris non traitées                   Souris traitées par Bioparyl

0    ....................    10/10    ....................    10/10
24   ....................    0/10     ....................    7/10
60   ....................    −        ....................    5/10
90   ....................    −        ....................    5/10

Bioparyl : 2 x 3mg/j pendant 8 jours consécutifs (voie i.p.)

EP 0 352 146 B1

Le tableau III présente l'inhibition de la multiplication des cellules cancéreuses chez la souris sous l'effet du Bioparyl (Lymphone YC8).

TABLEAU III

Survie de souris ♂ BALB/C porteuses du lymphome YC8 et traitées par Bioparyl

| Survie (jours) | Souris non traitées | Souris traitées par Bioparyl |
|---|---|---|
| 0 | 10/10 | 10/10 |
| 25 | 4/10 | 10/10 |
| 30 | 0/10 | 5/10 |
| 60 | - | 5/10 |
| 90 | - | 5/10 |

Bioparyl : 2 x 3 mg/j pendant 8 jours consécutifs (voie i.p.)

Survie des souris ♀ BALB/C porteuses du lymphome YC8 et traitées par Biopary

| Survie (jours) | Souris non traitées | Souris traitées par Bioparyl |
|---|---|---|
| 0 | 10/10 | 10/10 |
| 24 | 3/10 | 9/10 |
| 30 | 0/10 | 8/10 |
| 60 | - | 5/10 |
| 90 | - | 5/10 |

Bioparyl : 2 x 3mg/j pendant 8 jours consécutifs (voie i.p.)

## TOXICITE ET POSOLOGIE

Toxicité aigüe du Bioparyl : La préparation standardisée de Bioparyl administrée par voie orale à dose unique à des souris BALB C et Swiss ainsi qu'à des rats, s'est révélée non toxique. En effet, la DL50 chez les souris est supérieure à 2000 milligrammes par kilogramme. La DL50 chez le rat est supérieure à 3000 milligrammes par kilogramme.

Toxicité chronique : Le Bioparyl administré par voie orale aux doses de 2000 milligrammes par kilogramme deux fois par semaine pendant trois mois consécutifs ne modifie pas le comportement des souris qui ont une croissance pondérale normale et une prolificté inchangée. Une dose de 1000 milligrammes par jour (voie orale, ce qui correspond à la dose active journalière) pendant des mois est parfaitement bien tolérée par le sujet humain.

Toxicité aigüe par voie intra-péritonéale ou intraveineuse : (souris BALB C et Swiss). Le DL50 se situe vers 1200 milligrammes/kg et la dose tolérée est de 1000 milligrammes par kilogramme. 250 milligrammes/kg/jour pendant 2 mois sont parfaitement bien tolérés et le développement des souris est tout à fait normal. Une dose de Bioparyl de 250 milligrammes à 800 milligrammes par kilogramme , injectée par voie intraveineuse chez les souris Swiss est parfaitement tolérée. Chez l'homme , des doses orales de 1000 - 3000 milligrammes par jour et pendant des mois sont bien tolérées ; la voie intramusculaire peut être envisagée aux doses de 250 - 1000 milligrammes/jour et par voie intraveineuse 250 - 800 milligrammes par jour.

## Revendications

1. Une méthode d'obtention d'une substance à partir des feuilles dorées d'automne de l'arbre Ginko Biloba qui comprend:
   (1) une extraction par l'eau,
   (2) une concentration de l'extrait,
   (3) une hydrolyse acide,
   (4) un ajustement du pH à une valeur comprise entre 3 et 4,
   (5) une centrifugation pour ne récupérer que le surnageant,
   (6) une chromatographie sur colonne avec élution par l'eau et sélection des fractions ayant une activité inhibitrice de l'activité enzymatique de la ribonucléase, constituant ladite substance,
   (7) une concentration et un conditionnement.

2. Substance pouvant être obtenue par le procédé selon la revendication 1.

3. Composition pharmaceutique comportant la substance selon la revendication 2 et un support pharmaceutique acceptable.

4. Composition selon la revendication 3, sous forme d'unité de dosage.

5. Unité de dosage selon la revendication 4 contenant 0,1 à 3 grammes de ladite substance.

6. Utilisation d'une substance selon la revendication 2 pour l'obtention d'un médicament destiné à inhiber l'activité enzymatique de la ribonucléase d'un système biologique.

7. Utilisation d'une substance selon la revendication 2 pour l'obtention d'un médicament destiné à inhiber l'activité enzymatique de la ribonucléase d'un système biologique contenant des cellules cancéreuses ou virosées chez les mammifères.

8. Utilisation d'une substance selon la revendication 2 pour l'obtention d'un médicament destiné à inhiber l'activité enzymatique de la ribonucléase d'un système biologique contenant des cellules en dysfonctions que le fer suractive.

9. Utilisation d'une substance selon la revendication 2 pour l'obtention d'un médicament destiné à inhiber l'activité enzymatique de la ribonucléase d'un système biologique contenant des cellules cancéreuses ou virosées chez les mammifères, et destiné à ralentir la formation de blastes chez les sujets leucémiques.

10. Utilisation d'une substance selon la revendication 2 pour l'obtention d'un médicament destiné à inhiber l'activité enzymatique de la ribonucléase d'un système biologique contenant des cellules cancéreuses ou virosées chez les mammifères, et destiné à freiner le développement des cellules cancéreuses.

11. Utilisation d'une substance selon la revendication 2 pour l'obtention d'un médicament destiné à traiter des

EP 0 352 146 B1

mammifères développant une fibrose de certains tissus (suite à un traitement physique, chimique ou sans cause connue ).

12. Utilisation d'une substance selon la revendication 2 pour l'obtention d'un médicament destiné à éviter le développement d'une fibrose de certains tissus (suite à un traitement physique, chimique ou sans cause connue).

13. Utilisation d'une substance selon la revendication 2 pour l'obtention d'un médicament destiné à supprimer une fibrose installée de certains tissus (suite à un traitement physique, chimique ou sans cause connue).

14. Utilisation d'une substance selon la revendication 2 pour l'obtention d'un médicament destiné à réduire les taux élevés des gamma-globulines ou des immuno-globulines chez les mammifères.

**Patentansprüche**

1. Methode zum Erhalt einer Substanz ausgehend von den goldfarbenen Herbstblättern des Gingko Biloba Baums, die besteht in:
   (1) einer Extraktion durch Wasser,
   (2) einer Konzentrierung des Extrakts,
   (3) einer sauren Hydrolyse,
   (4) einer Anpassung des pH-Werts an einen Wert zwischen 3 und 4,
   (5) einer Zentrifugierung, um nur das Aufschwimmende zu gewinnen,
   (6) einer Chromatographie auf Säule mit Elution durch Wasser und Auswahl der die Substanz bildenden Anteile, die hemmend auf die enzymatische Aktivität der Ribonuklease einwirken,
   (7) einer Konzentrierung und Aufbereitung.

2. Substanz, die durch das Verfahren nach Anspruch 1 erhalten werden kann.

3. Pharmazeutische Zusammensetzung, die die Substanz nach Anspruch 2 und einen akzeptablen pharmazeutischen Träger aufweist.

4. Zusammensetzung nach Anspruch 3 in Form einer Dosiereinheit.

5. Dosiereinheit nach Anspruch 4, die 0,1 bis 3 Gramm dieser Substanz enthält.

6. Verwendung einer Substanz nach Anspruch 2 zur Herstellung eines Medikaments, das die enzymatische Aktivität der Ribonuklease eines biologischen Systems hemmen soll.

7. Verwendung einer Substanz nach Anspruch 2 zur Herstellung eines Medikaments, das die enzymatische Aktivität der Ribonuklease eines biologischen Systems hemmen soll, das krebs- oder virusbefallene Zellen bei Säugetieren enthält.

8. Verwendung einer Substanz nach Anspruch 2 zur Herstellung eines Medikaments, das die enzymatische Aktivität der Ribonuklease eines biologischen Systems hemmen soll, das funktionsgestörte Zellen enthält, die das Eisen überaktiviert.

9. Verwendung einer Substanz nach Anspruch 2 zur Herstellung eines Medikaments, das die enzymatische Aktivität der Ribonuklease eines biologischen Systems hemmen soll, das krebs- oder virusbefallene Zellen bei Säugetieren enthält, und das die Bildung von Keimen bei Leukämiekranken verlangsamen soll.

10. Verwendung einer Substanz nach Anspruch 2 zur Herstellung eines Medikaments, das die enzymatische Aktivität der Ribonuklease eines biologischen Systems hemmen soll, das krebs- oder virusbefallene Zellen bei Säugetieren enthält und dazu bestimmt ist, die Entwicklung von Krebszellen zu bremsen.

11. Verwendung einer Substanz nach Anspruch 2 zur Herstellung eines Medikaments zur Behandlung von Säugetieren, die eine Fibrosis gewisser Gewebe entwickeln (nach einer physikalischen oder chemischen Behandlung oder ohne bekannten Grund).

12. Verwendung einer Substanz nach Anspruch 2 zur Herstellung eines Medikaments, das die Entwicklung

12

einer Fibrosis gewisser Gewebe vermeiden soll (nach einer physikalischen oder chemischen Behandlung oder ohne bekannten Grund).

13. Verwendung einer Substanz nach Anspruch 2 zur Herstellung eines Medikaments, das eine vorhandene Fibrosis gewisser Gewebe beseitigen soll (nach einer physikalischen oder chemischen Behandlung oder ohne bekannten Grund).

14. Verwendung einer Substanz nach Anspruch 2 zur Herstellung eines Medikaments, das die hohen Anteile von Gamma-Globulinen oder Immuno-Globulinen bei Säugetieren reduzieren soll.

**Claims**

1. A method of obtaining a substance from golden autumn leaves of the Gingko biloba tree, which comprises
   (1) extraction with water,
   (2) concentration of the extract,
   (3) acid hydrolysis,
   (4) adjusting the pH to a value between 3 and 4,
   (5) centrifuging and recovering the supernatant only,
   (6) column chromatography, elution with water and selection of certain fractions that have an inhibiting activity on the enzyme activity of ribonuclease, said fractions constituting said subtance,
   (7) concentration and conditioning.

2. A substance being obtainable with the method of claim 1.

3. A pharmaceutical composition comprising a substance according to claim 2 and a pharmaceutically acceptable carrier.

4. A composition according to claim 3 in an unit dosage form.

5. A unit dosage according to claim 4, containing 0,1 to 3 g of said substance.

6. Use of a substance according to claim 2 for obtaining a drug destinated to inhibit the enzyme activity of ribonuclease of a biological system.

7. Use of a substance according to claim 2 for obtaining a drug destinated to inhibit the enzyme activity of ribonuclease of a biological system containing cancer or viral cells in mammals.

8. Use of a substance according to claim 2 for obtaining a drug destinated to inhibit the enzyme activity of ribonuclease of a biological system containing dysfunctional cells that are over-stimulated by iron.

9. Use of a substance according to claim 2 for obtaining a drug destinated to inhibit the enzyme activity of ribonuclease of a biological system containing cancer or viral cells in mammals and destinated to slow-down the formation of blasts in leukemic subjects.

10. Use of a substance according to claim 2 for obtaining a drug destinated to inhibit the enzyme activity of ribonuclease of a biological system containing cancer or viral cells in mammals and destinated to restrain the development of cancer cells.

11. Use of substance according to claim 2 for obtaining a drug destinated to treat mammals developping fibrosis of certain tissues (consequent to a physical, chemical treatment or without known cause).

12. Use of substance according to claim 2 for obtaining a drug destinated to prevent the development of a fibrosis of certain tissues (consequent to a physical, chemical treatment or without known cause).

13. Use of substance according to claim 2 for obtaining a drug destinated to suppress settled down fibrosis of certain tissues (after a physical treatment or without known cause).

14. Use of a substance according to claim 2 for obtaining a drug destinated to reduce the high levels of gamma-globulines or immuno-globulines in mammals.

Figure 1

Figure 2

Bioparyl (μg/essai)
0,02 ml de plasma dilué (1/10)
sujet : aplasie medullaire

Bioparyl (μg/essai)
0,02 ml de plasma dilué (1/10)
sujet : thrombopenie

Bioparyl (μg/essai)
0,02 ml de plasma dilué (1/10)
sujet : polyarthrite chronique

Bioparyl (μg/essai)
0,02 ml de plasma dilué (1/10)
sujet : cancer de la thyroide

Figure 3

ARN-r
ARN 4S

Bioparyl (µg/essai)
0,02 ml de plasma dilué (1/10)
(cancer du pancreas )

Bioparyl (µg/essai)
0,02 ml de plasma dilué (1/10)
( Leucémie myéloide chronique )

EP 0 352 146 B1

Figure 4

Bioparyl (µg/essai)

0.02 ml de plasma dilué (1/10)

sujet sain

Figure 5

Figure 6

ml de plasma dilué (1/10)
sujet : cancer du sein,récidive

ml de plasma dilué (1/10)

sujet : leucémie myéloide chronique

Figure 7

Figure 8

EP 0 352 146 B1

Figure 9

22